# EUROPEAN PATENT APPLICATION

(11) **EP 0 680 740 A1**
(43) Date of publication of application: **08.11.1995**
(21) Application number: 94107190.4
(22) Date of filing: 07.05.1994
(51) Int. Cl.: A61F 13/58

(54) **Separation aid for release paper of a fastening system of a sanitary article**

(71) Applicant: THE PROCTER & GAMBLE COMPANY, Cincinnati, Ohio 45202 (US)
(72) Inventor: Thurnay, Eva Susanne Dominique, D-60489 Frankfurt (DE)
(74) Representative: Bottema, Johan Jan

(57) **Abstract**

The present invention relates to absorbent products which are used in combination with undergarments. Such products are for example sanitary napkins, catamenials or panty liners (1) and they have a fastening adhesive to attach them to the undergarment. According to the invention the fastening adhesive is covered by a protective cover means (10) extending up to the periphery of the adhesive. In order to separate the cover means from the absorbent product a release aid for example in the form of a two-part cover means (14,16) is included.

## Description

### Field of the invention

The present invention relates to absorbent products like for example sanitary napkins, catamenials or panty liners which are used in combination with undergarments. These absorbent products have a fastening adhesive to attach them to the undergarment. The fastening adhesive is covered by a protective cover means extending up to the periphery of the adhesive. In order to separate the cover means from the absorbent product a release aid for example in the form of a two-part cover means is included.

### Background of the invention

Fastening adhesives for sanitary napkins, catamenials or panty liners are well-known in the art. Usually, they cover part of the garment facing side of these products. The adhesive allows improved fixation of the products to the undergarment when applying the product and during use. In order to protect the adhesive prior to usage of the product it is common to overlay the adhesive with a protective cover means most often a release paper. Typically these protective cover means extend beyond the periphery of the adhesive area formed by the panty fastening adhesive to allow easy delamination from the absorbent product by providing an unadhered edge which can be easily grasped by the user when trying to separate the cover means from the product.

This delamination aid by extending the cover means beyond the periphery of the adhesive area naturally involves additional material consumption and the associated cost and disposability drawbacks. Also it becomes difficult to provide this extending delamination aid for adhesive areas having a periphery which is co-extensive with the absorbent product itself. For example, EP-A-0 237 786 discloses full surface panty fastening adhesive covered by release paper.

However, this disclosure does not indicate how to separate the release paper or that this may be difficult.

It is known in the art of adhesive stickers, like for example bumper stickers, to have the release paper split into two or several parts in order to facilitate delamination prior to use. However, this has never been suggested as a usable feature for absorbent products having adhesive areas on their garment facing side.

### Brief description of the drawings

Figure 1 shows a plan view of the garment side of an absorbent article comprising the present invention.

Figure 2 shows the article of Figure 1 directly before use of the present invention.

Figure 3 shows an embodiment of an article according to the invention having wings.

Figure 4 shows an alternative embodiment of the present invention having wings with three separate applications of the invention.

### Brief description of the invention

The present invention relates to disposable absorbent articles having a body facing surface and a garment facing surface and an adhesive area on the garment facing surface. A cover means to protect the adhesive area prior to use is provided extending essentially up to the periphery of the adhesive area and comprising a release aid. The release aid is provided by separating the cover means into more than one portion along a dividing line.

In a preferred embodiment of the invention, the release aid separates the cover means into two portions and the dividing line is preferably not parallel to the longitudinal axis of the absorbent product.

If the cover means is separated along two dividing lines, three or four portions of the cover means may be formed. In this case it is preferable that both lines are essentially parallel to the longitudinal axis of the absorbent article thereby separating the cover means into a central and two outer portions.

Especially if the article is a sanitary napkin, catamenial or panty liner having wings it is desired to have the release aid being formed by two dividing lines essentially parallel to the longitudinal axis of the article, such that the cover means is split into portions two of which are co-extensive with the wings while the third "center" portion is co-extensive with the remainder parts of the article.

In an alternative embodiment the cover means may comprise portions which overlap along a dividing line.

### Detailed description of the invention

According to the present invention an absorbent article for use in combination with an undergarment has an adhesive for attachment of the article to the undergarment. The article comprises a cover means covering the adhesive and the cover means is released prior to use of the article.

Any absorbent article regardless of material or functional composition is susceptible to the present invention provided an adhesive area on the garment facing side is covered by a cover means. In particular, sanitary napkins, catamenials and panty liners whether used for incontinence discharges or menstrual or other discharges are considered to be susceptible to the present invention. A typical absorbent article susceptible to the present invention is a panty liner having a thin absorbent structure overlaid on the body facing side by a liquid permeable topsheet and overlaid on the garment facing side by a liquid impermeable backsheet. Optionally, the article may comprise wings or flaps laterally extending from the central portion of the article away from the longitudinal axis and folded around the crotch portion of an undergarment during use. Details on the articles contemplated in combination with the present invention can be found in many patent disclosures and include those commercially available.

The article comprises an adhesive area on its garment facing surface which adhesive is protected by a cover means which cover means is released prior to use of the article. The adhesive area need not be, but preferably is, co-extensive with the garment facing surface of the article. Also the adhesive area need not be fully covered by adhesive but may for example be comprised of homogeniously distributed adhesive filaments leaving small areas uncovered between the filaments. Alternatively an adhesive area of which a central part is not covered by adhesive may be formed. Preferably however, the adhesive area is covered with adhesive essentially throughout the area inside its periphery.

The adhesive area is covered by the cover means such that the cover means extends substantially to the periphery of the adhesive area. Therefore it preferably extends to the periphery of the absorbent articles's garment facing surface which is preferably covered by adhesive as indicated above.

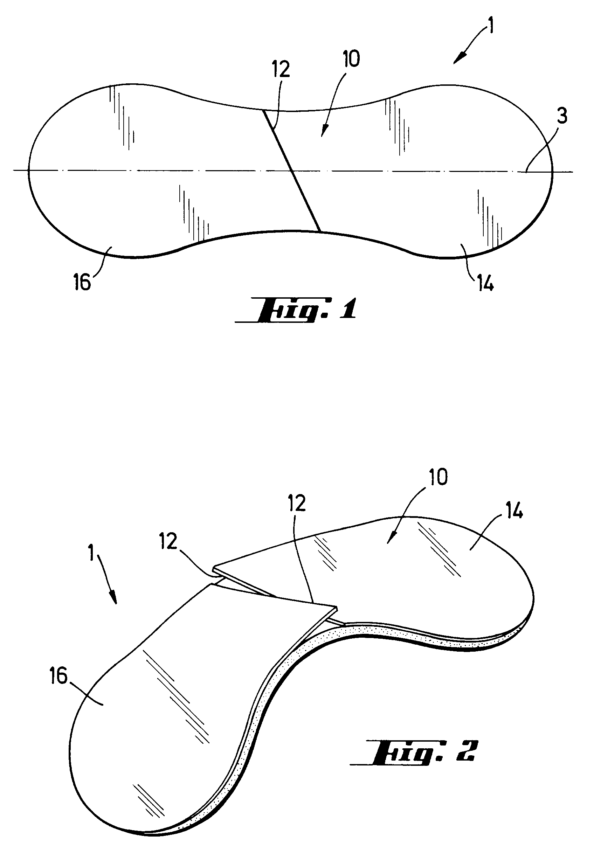

In order to aid release of the cover means from the adhesive the cover means comprises a dividing line separating the cover means into more than one portion. As shown in Figure 1 an embodiment of the present invention is a panty liner (1) having a longitudinal axis (3) and a cover means (10) covering the adhesive area and fully covering the garment facing surface of the sanitary napkin (1). The cover means (10) is divided by a dividing line (12) into a first cover means portion (14) and a second cover means portion (16).

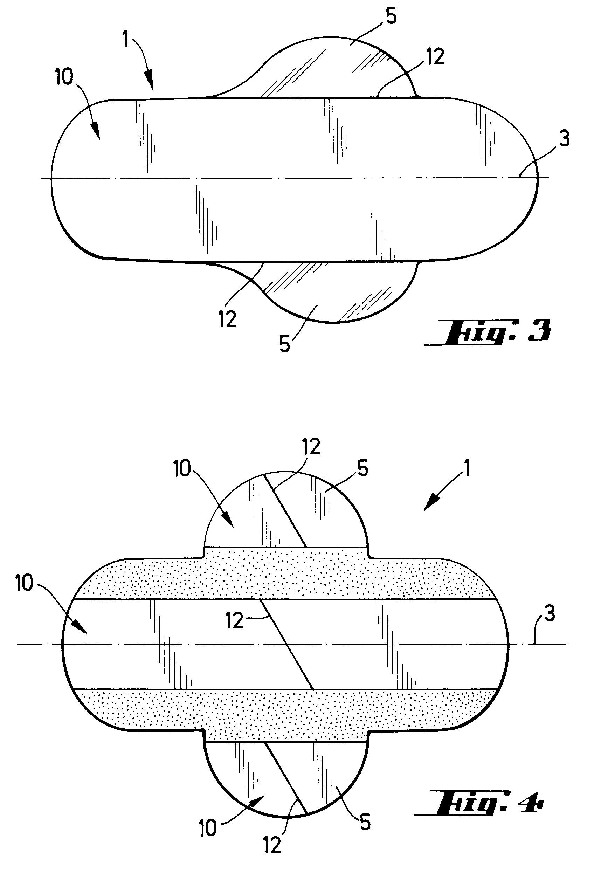

Figure 2 shows the use of the release aid of Figure 1 by bending the article (1) such that the first and second portion of the cover means fold up allowing easy handling and release of the cover means.

Figure 3 shows an alternative embodiment in that the absorbent article has wings (5) and two dividing lines (12) thereby creating three portions of the cover means. Two portions are essentially co-extensive with the wings and the central portion is essentially co-extensive with the remainder of the article (1).

In Figure 4 an alternative embodiment of the present invention is shown having three cover means (10), each of which is split by one dividing line (12) and which are placed individually and not connected to each other on the wings and in the central part of the article (1).

The adhesive for fastening the article (1) in an undergarment can be any adhesive useful for the desired application. Usually it is a pressure sensitive adhesive which can be applied in any way usual in the art. Particularly, the adhesive can be coated as hot melt or cold solution, either to the garment facing surface of the article or to the cover means which then carries it to the article. Necessarily, the adhesion force of the adhesive to the undergarment facing surface of the article needs to be larger or equal to that adhesion force which it has to the cover means. Also it is desirable to have an adhesive which does not separate during use from the article and leaves adhesive traces in the undergarment when separating the article from the undergarment.

The cover means can be provided by any usual material in the art, particularly by paper or film of natural or synthetic origin such as for example cellulose, polyethylene, polypropylene, copolymers of different monomers and other materials. Typically, paper comprising new and/or recycled fibers can be used.

To satisfy the requirements of adhesion force relation between cover means/adhesive and garment facing side surface of the article/adhesive it is desirable to use a cover means treated with an anti-adhesion material on the side where the adhesive is contacted. Such anti-adhesion materials typically comprise silicon or other waxy type of anti-adhesion materials.

Particularly preferred as the cover means are paper sheets coated with silicon on one or both sides. If such papers are used a particular advantage of them is that the adhesive can be applied to the cover means and the cover means together with the adhesive can then be applied to the absorbent article. This allows the use of adhesives which have an application temperature or other characteristic which is unsuitable for applying them directly to the garment facing side of the article while applying them to the cover means has no disadvantageous effect.

It will be appreciated that the above explanations and embodiments are disclosed for the purpose of illustration and enablement and are not to be construed as limiting the present invention which is defined in the claims.

## Claims

1. Disposable absorbent article having a body facing surface and a garment facing surface and further having a longitudinal axis, said article comprising:
- an adhesive area on said garment facing surface; and
- a cover means for said adhesive area extending essentially up to the periphery of said adhesive area; said cover means having a release aid, said release aid separating said cover means into more than one portion along a dividing line.

2. A disposable absorbent article according to Claim 1 in which said cover means has two portions.

3. A disposable absorbent article according to Claim 2 in which said dividing line is not parallel to said longitudinal axis.

4. A disposable absorbent article according to Claim 1 wherein said release aid separates said cover means along two dividing lines, said dividing lines being essentially parallel to said longitudinal axis, creating two outer portions and one center portion of said cover means.

5. A disposable absorbent article according to any of the preceding Claims wherein said article is an adult incontinence insert, a catamenial, a sanitary napkin or a pantyliner, preferably having wings.

6. A disposable absorbent article according to Claim 4 or 5 wherein said article comprises wings and said cover means has two dividing lines which are essentially parallel to said longitudinal axis and which create two outer portions of said cover means and said outer portions being essentially co-extensive with said wings.

7. A disposable absorbent article according to any of the preceding Claims wherein the cover means is a release paper.

8. A disposable absorbent article according to any of the preceding Claims wherein said adhesive area is covered by adhesive homogeniously inside said periphery of said adhesive area.

9. A disposable absorbent article according to any of the preceding Claims wherein said adhesive area is co-extensive with said garment facing surface.

10. A disposable absorbent article according to any of the preceding Claims wherein said portions of the cover means overlap along a dividing line.
